# EUROPEAN PATENT SPECIFICATION

(11) **EP 1 882 489 B1**
(45) Date of publication and mention of the grant of the patent: **22.09.2010**
(21) Application number: 07014087.6
(22) Date of filing: 18.07.2007
(51) Int. Cl.: A61M 25/10

(54) **Fluid-supplying/discharging system for use in medical apparatuses and endoscope apparatuses**
Flüssigkeitszufuhr- und -auslasssystem zur Verwendung in medizinischen Vorrichtungen sowie in endoskopischen Vorrichtungen
Système d'alimentation/évacuation de fluides à utiliser dans des appareils médicaux et des appareils endoscopiques

(30) Priority: 25.07.2006 JP 2006202341
(43) Date of publication of application: 30.01.2008
(73) Proprietor: OLYMPUS MEDICAL SYSTEMS CORP., Shibuya-ku Tokyo 151-0072 (JP)
(72) Inventor: Matsui, Raifu, Hachioji-shi Tokyo 192-8512 (JP); Matsuura, Noboyuki, Hachioji-shi Tokyo 192-8512 (JP); Takase, Seisuke, Hachioji-shi Tokyo 192-8512 (JP); Kimura, Hidenobu, Hachioji-shi Tokyo 192-8512 (JP); Yoshida, Takatoshi, Hachioji-shi Tokyo 192-8512 (JP)
(74) Representative: von Hellfeld, Axel

(56) References cited:
- WO-A-03/054365
- WO-A-2005/089625
- JP-A- 2002 301 019
- JP-A- 2005 220 810
- US-B1- 6 419 657

## Description

The present invention relates to a fluid-supplying/discharging system for use in medical apparatuses, which can supply and discharge a fluid into and from a balloon provided in a tube, such as an overtube, or a balloon provided in the insertion section of an endoscope. The present invention also relates to an endoscope apparatus including the fluid-supplying/discharging system.

In controlling the balloon provided in an overtube or the like, which is used in combination with an endoscope, the balloon is inflated slowly. This is because the body wall, e.g., the thin tubular organ such as the small intestine, must be so slowly expanded as to minimize the adverse influence on the body wall. On the other hand, when the balloon needs to be deflated, it is preferably deflated as fast as possible so that the doctor may quickly start performing the next medical step.

As disclosed in, for example, Jpn. Pat. Appln. KOKAI Publication No. 2002-301019, a system is known, which can change the operating speed of the drive motor in a fluid-supplying/discharging apparatus to change the rate at which a fluid is supplied into, or discharge the fluid from, a balloon.

As is described in Publication No. 2002-301019, however, a complex electrical control must be performed on the motor in order to change the rotation speed of the motor. When the balloon is inflated or deflated at high speed or low speed, it should be stably controlled. Having a control device and a pump that can accomplish such stable control of the balloon, it is inevitable that the fluid-supplying/discharging apparatus will incur a high manufacturing cost.

US 6,419,657 B1 relates to an apparatus for regulating flow of an inflation fluid to a stent delivery balloon. A valve, which is situated in the flow path of the inflation lumen, regulates the flow of liquid used to inflate and deflate the balloon. The valve performs to restrict flow and to respond to pressure applied to assure a desired inflation rate when fluid is flowing toward the delivery balloon. On deflation, when fluid is flowing away from the delivery balloon, the valve is designed to offer little or no resistance, allowing the balloon to be deflated quickly.

The present invention has been made to solve the problem described above. An object of the invention is to provide a fluid-supplying/discharging system for use in medical apparatuses, which can easily control the speed at which a balloon can be inflated and deflated, and to provide an endoscope apparatus that has a fluid-supplying/discharging system for use in medical apparatuses.

A fluid-supplying/discharging system for use in medical devices according to this invention is defined by the independent claims. Preferred embodiments of the fluid-supplying/discharching system for use in medical devices according to the present invention are defined by the dependent claims.

The invention can be more fully understood from the following detailed description when taken in conjunction with the accompanying drawings, in which:
FIG. 1 is a schematic diagram showing an endoscope apparatus according to a first embodiment of the present invention;
FIG. 2A is a partially sectional view showing the overtube provided in the endoscope apparatus according to the first embodiment;
FIG. 2B is a schematic diagram showing a fluid-supplying/discharging system that is connected to the overtube of the endoscope apparatus according to the first embodiment;
FIG. 3A is a schematic perspective view of a tubular-path-resistance changing mechanism according to the first embodiment, showing a tubular path unpressed;
FIG. 3B is a schematic perspective view of the tubular-path-resistance changing mechanism according to the first embodiment, showing the tubular path depressed;
FIG. 4A is a flowchart explaining how a signals is supplied and how the fluid-supplying/discharging system operates, when the pressurizing button is pushed at the remote controller provided on the endoscope apparatus according to the first embodiment;
FIG. 4B is a flowchart explaining how a signal is supplied and how the fluid-supplying/discharging system operates, when the stop button is pushed at the remote controller provided on the endoscope apparatus according to the first embodiment;
FIG. 4C is a flowchart explaining how a signal is supplied and how the fluid-supplying/discharging system operates, when the depressurizing button is pushed at the remote controller provided on the endoscope apparatus according to the first embodiment;
FIG. 5 is a schematic diagram showing the insertion section and overtube of the endoscope apparatus according to the first embodiment, both inserted in the small intestine;
FIG. 6 is a schematic diagram showing the insertion section and overtube of the endoscope apparatus according to the first embodiment, both inserted in the small intestine, and showing the balloon provided on the overtube, which is inflated and holds the overtube at a specific position in the small intestine;
FIG. 7 is a schematic diagram explaining how the distal end of the insertion section of the endoscope of the endoscope apparatus according to the first embodiment is moved deeper in the small intestine, while the insertion section and overtube of the endoscope apparatus remain in the small intestine and the balloon remains inflated, holding the overtube at the specific position in the small intestine;
FIG. 8 is a schematic diagram explaining how the overtube is moved deeper in the small intestine along the insertion section of the endoscope according to the first embodiment, while the insertion section of the endoscope apparatus and the overtube remain in the small intestine;
FIG. 9 is a schematic diagram showing the insertion section and overtube of the endoscope apparatus according to the first embodiment, which have been inserted deeper in the small intestine than is shown in FIG. 5;
FIG. 10 is a schematic diagram of the insertion section and overtube of the endoscope apparatus according to the first embodiment, both inserted in the small intestine, showing the balloon provided on the overtube, which is inflated and holds the overtube at a specific position in the small intestine;
FIG. 11A is a partially sectional view showing the overtube of an endoscope apparatus according to a second embodiment of the present invention;
FIG. 11B is a fluid-supplying/discharging system to be connected to the overtube of the endoscope apparatus according to the second embodiment;
FIG. 12A is a schematic perspective view showing the tubular-path-resistance changing mechanism used in the fluid-supplying/discharging system provided in the endoscope apparatus according to the second embodiment;
FIG. 12B is a sectional view taken along line 12B-12B shown in FIG. 12A, showing the tubular-path-resistance changing mechanism provided in the endoscope apparatus according to the second embodiment;
FIG. 13A is a schematic diagram showing the fluid-supplying/discharging system provided in an endoscope apparatus according to a third embodiment of the present invention;
FIG. 13B is a longitudinal sectional view showing the tubular-path-resistance changing mechanism used in the fluid-supplying/discharging system provided in the endoscope apparatus according to the third embodiment;
FIG. 13C is a magnified sectional view showing the movable body incorporated in the tubular-path-resistance changing mechanism shown in FIG. 13B, which is used in the fluid-supplying/discharging system provided in an endoscope apparatus according to the third embodiment;
FIG. 14A is a longitudinal sectional view showing the tubular-path-resistance changing mechanism used in the fluid-supplying/discharging system provided in an endoscope apparatus according to an example useful for understanding this invention;
FIG. 14B is another longitudinal sectional view showing the tubular-path-resistance changing mechanism of the fluid-supplying/discharging system provided in an endoscope apparatus according to the example useful for understanding this invention;
FIG. 14C is a schematic sectional view taken along line 14C-14C shown in FIG. 14A, showing the rotatable filter arranged in a space provided in the tubular-path-resistance changing mechanism of the fluid-supplying/discharging system provided in the endoscope apparatus according to the example; and
FIG. 15 is a schematic diagram showing an endoscope apparatus according to a fourth embodiment of the present invention.

Some of the best modes of the present invention will be described, with reference to the accompanying drawings.

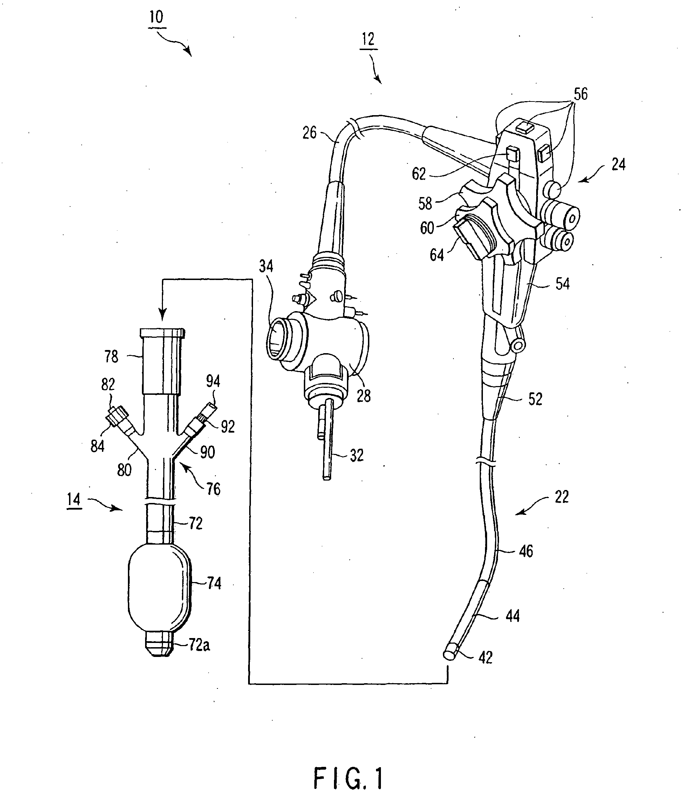

The first embodiment of the invention will be described with reference to FIGS. 1 to 10.

As shown in FIGS. 1 to 2B, an endoscope apparatus 10 according to a first embodiment includes an endoscope 12, an overtube (medical device) 14, and a fluid-supplying/discharging system 16 for use in medical apparatuses.

As FIG. 1 shows, the endoscope 12 includes an elongated insertion section 22 and an operation section 24 connected to the proximal end of the insertion section 22. A universal cable 26 is connected, at one end, to the proximal end of the operation section 24. The universal cable 26 can apply illumination light from a light source (not shown) and supply various signals. The other end of the universal cable 26 is provided on a connector unit 28. The connector unit 28 has a light guide connector 32 and an electrical connector 34. The light guide connector 32 is arranged coaxial with the universal cable 26. The light source mentioned above is connected to the light guide connector 32. To the electrical connector 34, a camera cable is connected, which can connect the connector 34 to a camera control unit (not shown).

The camera control unit is connected to a monitor (not shown). Thus, the camera control unit can process the video signal representing any optical image of the tissue or organ being examined, which has been formed by a solid-state imaging element such as CCD that will be described later. Using the signal thus processed, the monitor displays the image of the tissue or organ being examined.

The insertion section 22 includes a distal end part 42, a flexible part 44, and a flexible tubular part 46. The distal end part 42 is rigid. The flexible part 44 can bend up and down, left to right and vice versa. The flexible tubular part 46 is long and flexible.

The distal end part 42 is the most distal portion of the insertion section 22. The distal end part 42 incorporates an illumination optical system and an observation optical system including a solid-state imaging element, and has a forceps port (not shown) and a nozzle (not shown, either). The forceps port communicates with the instrument insertion channel (not shown). The nozzle is provided to supply air into body cavities and water to the observation lens. The instrument insertion channel communicates with the instrument insertion port (not shown) of the operation section 24.

The flexible part 44 is coupled, at distal end, to the proximal end of the distal end part 42. The flexible tubular part 46 is coupled, at distal end, to the proximal end of the flexible part 44. The operation section 24 is coupled, at distal end, to the proximal end of the flexible tubular part 46. Thus, the distal end of the operation section 24 is coupled to the proximal end of the insertion section 22.

The operation section 24 has a support part 52 at the distal end. The support part 52 supports the proximal end of the flexible tubular part 46. The support part 52 has a distal end, which is tapered, gradually narrower toward the proximal end of the flexible tubular part 46. The support part 52 has a grip 54 at its proximal end. The doctor may hold the grip 54 when he or she uses the operation section 24. The grip 54 has remote-control switches 56, which the doctor may operate to remote-control a video recording device (not shown), such as a VTR, and the camera control unit (not shown, either).

At the proximal end of the grip 54, flexible-part operating levers 58 and 60 are provided. The doctor may rotate these levers 58 and 60 in order to bend the flexible part 44, deviating from the axis of the flexible tubular part 46, or bending the flexible part 44 up or down, and to the left or the right. The lever 58 bents the flexible part 44 up when rotated in one direction, and down when operated in the other direction. Similarly, the lever 60 bends the flexible part 44 to the left when rotated in one direction, and to the right when rotated in the other direction.

A position-setting lever 62 is provided adjacent to the flexible-part operating lever 58. This lever 62 may be operated to retain the lever 58 at a desired position, thereby setting the flexible part 44 in a desired bent state. The lever 62 may be operated to release the flexible part 44 from that bent state. In other words, the lever 62 is operated to set and release the flexible part 44 in and from a desired bent state.

Like the flexible-part operating lever 58, the other flexible-part operating lever 60 has a position-setting lever 64. This lever 64 may be operated to release the flexible part 44 from a desired bent state. That is, the lever 64 is operated to set the flexible part 44 in a desired bent state and to release the part 44 from the bent state, allowing the flexible-part operating lever 60 to rotate.

The overtube 14 shown in FIG. 1 is mounted on a part of the insertion section 22 and is used in order to facilitate the insertion of the insertion section 22 of the endoscope 12.

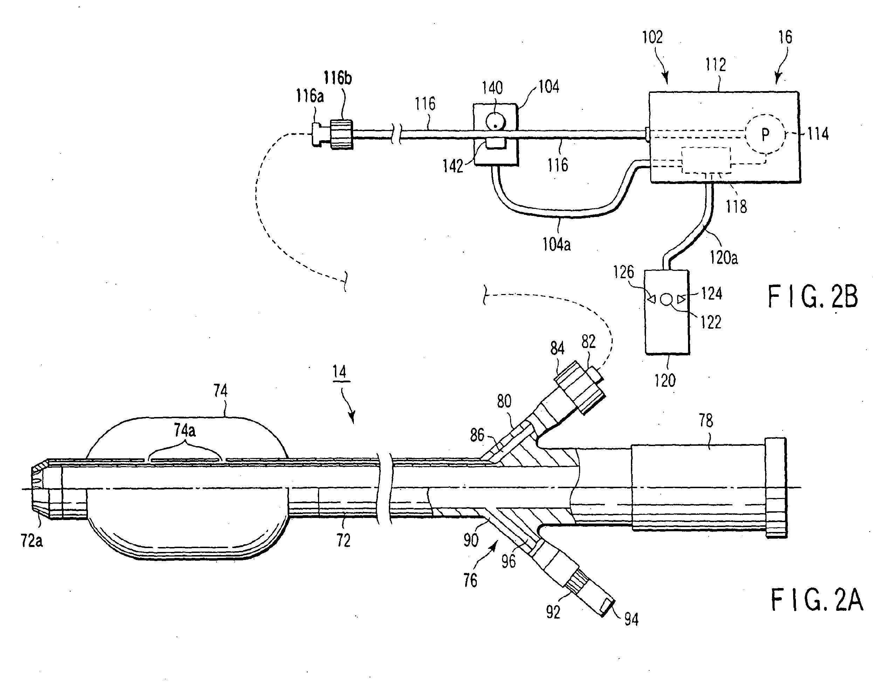

As shown in FIG. 2A, the overtube 14, which can be removably coupled to the insertion section 22 of the endoscope 12, includes a tubular body 72, a balloon 74, a branched connecting part 76, and a proximal-side grip 78. The tubular body 72 is an elongate hollow cylinder. The balloon 74 can be inflated and deflated. The tubular body 72 has a passage through which the insertion section 22 of the endoscope 12 can pass through (or be inserted). The tubular body 72 is flexible, as the flexible tubular part 46 of the insertion section 22 of the endoscope 12. Therefore, as the flexible tubular part 46 of the insertion section 22 is bent upon receiving a force from the body wall, the tubular body 72 is bent in conformity with the flexible tubular part 46 of the insertion section 22.

The balloon 74 is mounted on that part of the tubular body 72 which is near the distal end of the tubular body 72. The tubular body 72 has a distal tip 72a that is opaque to X rays. The proximal-side grip 78 is provided on the proximal end of the tubular body 72. The proximal-side grip 78 is made hard enough to be held well.

The branched connecting part 76 projects from the distal end of the proximal-side grip 78 and is located near the proximal end of the tubular body 72. The branched connecting part 76 has first and second rigid portions 80 and 90. The rigid portions 80 and 90 project from appropriate parts (extension bases) of the proximal end of the overtube 14 toward the proximal end of the proximal-side grip 78. Thus, the first and second rigid portions 80 and 90 extend from the proximal part of the tubular body 72, deviating from the axis of the tubular body 72. The first and second rigid portions 80 and 90 are located symmetrical to each other with respect to the axis of the tubular body 72.

A first cap 82 is provided on the extending end of the first rigid part 80. A first connecting part 84 is mounted on the first cap 82.

The tubular body 72 has a first communication path (communication tubular path) 86. The first communication path 86 extends from the distal end of the tubular body 72 to the first rigid part 80. The path 86 communicates with the interior of the first cap 82. The first communication path 86 is made in the tubular body 72 and extends along the axis of the tubular body 72. That part of the tubular body 72, which is near the distal end of the first communication path 82, has a plurality of openings 74a. The openings 74a open to the outside of the tubular body 72 and to the interior of the balloon 74. Hence, gas can be supplied into the balloon 74 from the proximal end of the first communication path 86, thereby to inflate the balloon 74. The gas can of course be discharged to deflate the balloon 74.

As described above, the first connecting part 84 is mounted on the first cap 82. The first connecting part 84 is shaped like a hollow cylinder and has a flange that extends in a radial direction from the first cap 82 for a predetermined distance. The proximal end of the first rigid part 80 abuts on the flange of the first connecting part 84. That is, a space is defined between the first cap 82 and the first rigid part 84.

A second cap 92 is provided on the extending end of the second rigid part 90. A second connecting part 94 is mounted on the second cap 92. The tubular body 72 has a second communication path (communication tubular path) 96. The second communication path 96 extends from the tubular body 72 to the proximal end of the second rigid part 90. The second communication path 96 extends along the axis of the second rigid part 90. The second communication path 96 communicates, at one end, with the interior of the tubular body 72.

As FIG. 2B shows, the fluid-supplying/discharging system 16 includes a supplying/discharging device 102 and a tubular-path-resistance changing mechanism (path-cross-section area changing mechanism) 104.

The supplying/discharging device 102 includes a housing 112, a pump 114, a tubular path (communication path) 116, a control circuit 118, and a remote controller 120. The pump 114 can supply and discharge (draw) gas. The pump 114 supplies gas in a regular manner. In other words, the gas is supplied from the pump 114 at an almost constant flow rate (i.e., in a particular amount of gas per unit time) and at an almost constant speed. The pump 114 discharges the gas at a substantially constant rate, too. That is, the pump 114 discharges the gas at an almost constant flow rate, that is, at an almost constant speed. A tubular path 116 is connected, at one end, to the pump 114. A tube cap 116a that has a tube-connecting part 116b is arranged at the other end of the tubular path 116. The tube cap 116a is connected to the first cap 82 of the overtube 14 and can be disconnected from the first cap 82. The pump 114 is electrically connected to the control circuit 118. The control circuit 118 controls not only the pump 114, but also the tubular-path-resistance changing mechanism 104. The control circuit 118 controls the pump 114 in both the gas-supplying operation and the gas-discharging operation. The control circuit 118 controls the pump 114 to prevent the pressure in the tubular path 116 from rising over a predetermined value.

A cable 120a electrically connects the remote controller 120 to the control circuit 118. The controller 120 includes a stop button 122, a pressurizing button 124, and a depressurizing button 126. When pushed, the stop button 122 generates a signal, which is input to the control circuit 118. Upon receiving the signal, the control circuit 118 makes the pump 114 stops operating. When pushed, the pressurizing button 124 generates a signal, which is input to the control circuit 118. Upon receiving this signal, the control circuit 118 makes the pump 114 supply gas into the tubular path 116. When pushed, the depressurizing button 126 generates a signal, which is input to the control circuit 118. Upon receiving this signal, the control circuit 118 makes the pump 114 discharge the gas from the tubular path 116.

The tubular path 116 is made of, for example, silicone rubber. The tubular path 116 is elastic, having its diameter decreased when pressed from outside and increased to the initial value when released from the pressure.

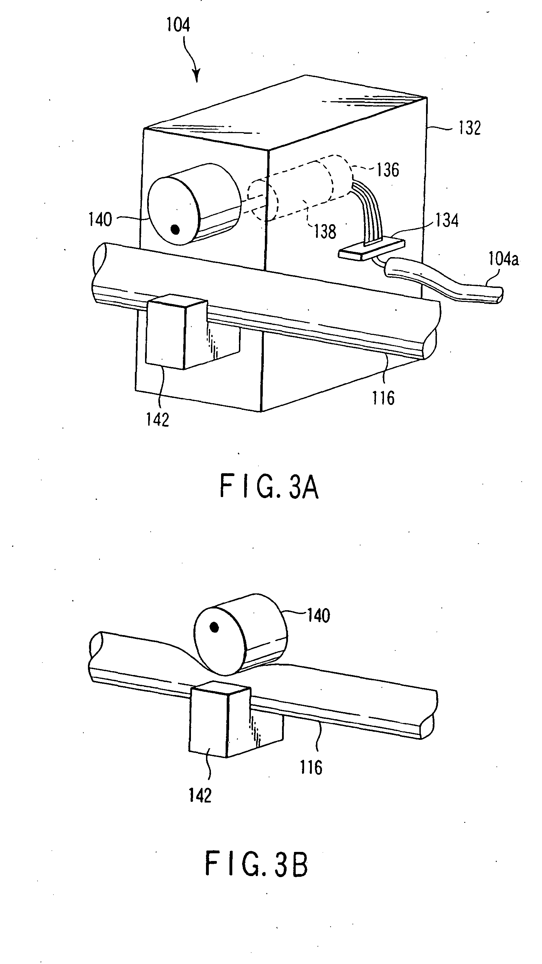

As shown in FIG. 3A, the tubular-path-resistance changing mechanism 104 includes a housing 132, an interface 134, an encoder 136, a motor 138, a pushing member (cam) 140, and a support member 142. The interface 134 is secured to one side of the housing 132. A cable 104a electrically connects the interface 134 to the control circuit 118. The encoder 136 and the motor 138 are secured in the housing 132 and are electrically connected to the control circuit 118 by the interface 134 and cable 104a. The encoder 136 detects the rotation speed of the driving shaft of the motor 138.

The pushing member 140 is arranged outside the housing 132 and mounted on a driving shaft of the motor 138. The pushing member 140 is therefore rotated when the motor 138 is driven. The support member 142 is secured to, and provided outside, the housing 132. The support member 142 has an L-shaped cross section, taken along a vertical plane, and holds the tubular path 116, jointly with the pushing member 140. The tubular path 116 can therefore be easily held in, and removed from, the gap between the pushing member 140 and the support member 142. While being used, the tubular path 116 is prevented from slipping from the gap between the pushing member 140 and the support member 142. The pushing member 140 has an elliptical cross section, taken along a plane perpendicular to its axis. The member 140 is so positioned that the driving shaft of the motor 138 passes the focus of the ellipse. The pushing member 140 stays in the position shown in FIG. 3B while the pump 114 is not being used.

As seen from FIG. 2B, the tubular-path-resistance changing mechanism 104 is a component provided independently of the supplying/discharging device 102. Nonetheless, the mechanism 104 and the device 102 may be formed integrally with each other, preferably.

How the endoscope apparatus 10 according to this embodiment operates will be explained.

First, the operation of the remote controller 120 will be explained.

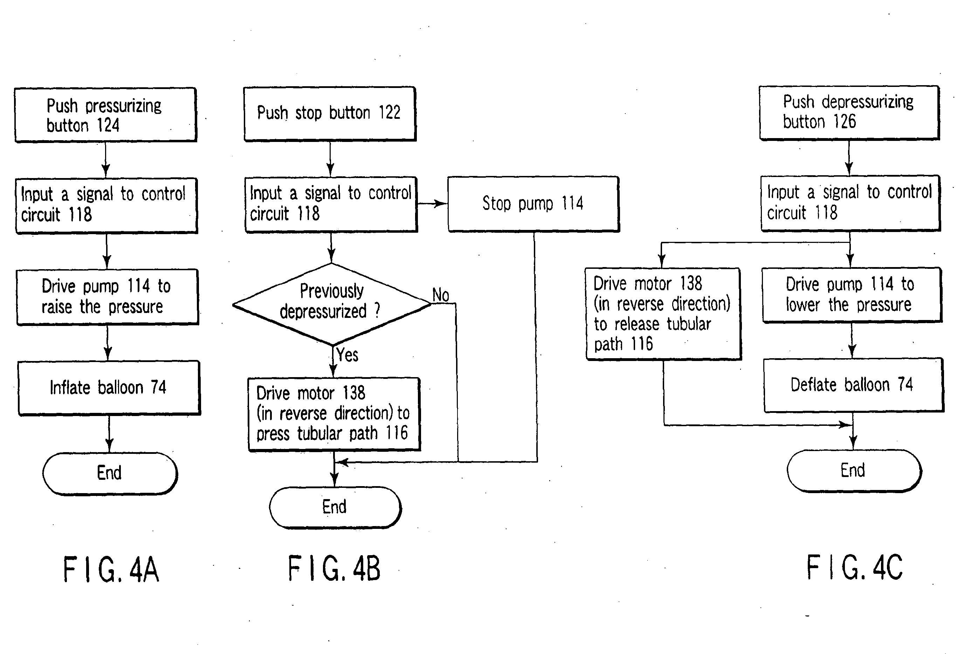

When the pressurizing button 124 is pushed, it generates a signal as is explained in FIG. 4A. The signal is supplied to the control circuit 118. Upon receiving the signal, the control circuit 118 drives the pump 114. As a result, gas is supplied into the balloon 74 through the tubular path 116 and the first communication path 86. The balloon 74 is thereby inflated.

As shown in FIG. 4B, when the stop button 122 is pushed, it generates a signal. The signal is supplied to the control circuit 118. Upon receiving the signal, the control circuit 118 stops driving the pump 114. At this point, it is determined which button, the pressurizing button 124 or the depressurizing button 126, has been pushed before the stop button 122 is pushed. If the pressurizing button 124 has been pushed before the stop button 122 (if the pressure has been raised previously), the operation is terminated. If the depressurizing button 126 has been pushed before the stop button 122 (if the pressure has been lowered previously), the motor 138 is driven, rotating the pushing member 140. The pushing member 140 caracoles around the axis of the driving shaft. In this case, the tubular path 116 is pushed from outside.

As shown in FIG. 4C, when the depressurizing button 126 is pushed, it generates a signal. This signal is supplied to the control circuit 118. Upon receiving the signal, the control circuit 118 drives the pump 114, which discharges gas, performing the function opposite to pressurizing. At the same time, the control circuit 118 drives the motor 138, whose driving shaft rotates the pushing member 140, half around the axis of the driving shaft. The tubular path 116 is no longer pressed. The gas is discharged from the balloon 74 through the first communication path 86 and tubular path 116. The balloon 74 is thereby deflated.

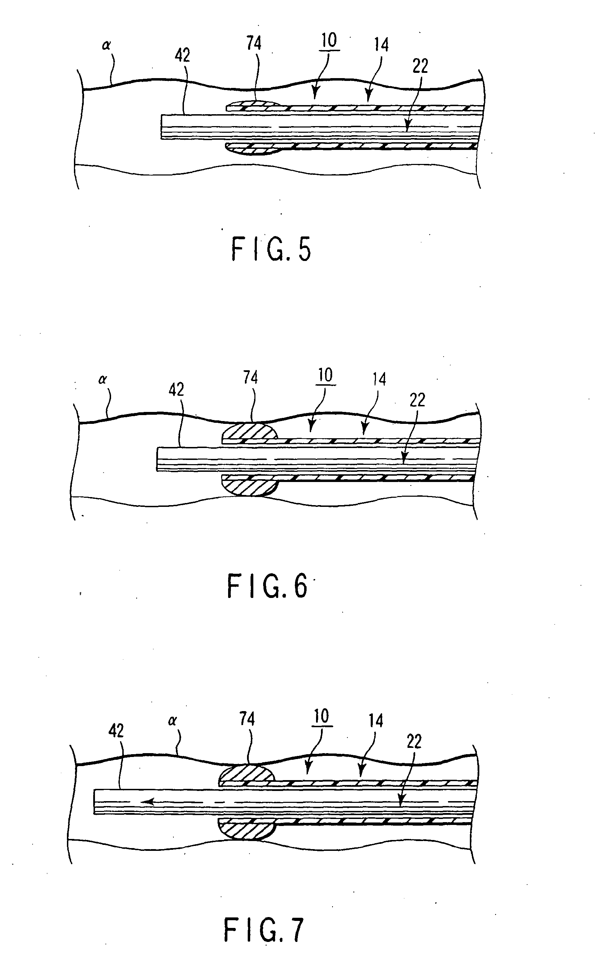

How the distal end of the insertion section 22 of the endoscope apparatus 12 is inserted deep into the small intestine α, whose wall is thinner than that of the large intestine, by using the overtube 14 will be explained with reference to FIGS. 5 to 10.

First, the overtube 14 is mounted on the insertion section 22 of the endoscope 12. Then, the overtube 14 is arranged close to the proximal end of the insertion section 22, as much as possible. Thus, the distal end of the insertion section 22 protrudes from the distal end of the overtube 14. The distal end portion of the insertion section 22 in this state is inserted through the subject's mouth into the small intestine α.

When the distal end of the insertion section 22 reaches a bending part of the small intestine α and can no longer be easily inserted deeper, the overtube 14 is moved along the insertion section 22 toward the distal end thereof (see FIG. 5). Then, the balloon 74 positioned near the distal end of the insertion section 22 is slowly inflated until it contacts the inner surface of the intestine wall (body wall) (see FIG. 6). As a result, the balloon 74 sets the overtube 14 in position with respect to the intestine wall.

To inflate the balloon 74, the doctor pushes the pressurizing button 124 provided on the remote controller 120 shown in FIG. 2B. When pushed, the pressurizing button 124 generates a signal, which is input from the remote controller 120 to the control circuit 118. Upon receiving the signal, the control circuit 118 drives the pump 114. The pump 114 supplies gas. At this time, the pushing member 140 presses the tubular path 116 in such a state as shown in FIG. 3B. That part of the tubular path 116 which is pressed by the member 140 is narrower than the other parts. The gas being supplied to the balloon 74 by the pump 114 operating in normal state meets a resistance in the narrower part of the tubular path 116. In other words, the gas cannot smoothly flow through the narrower part of the tubular path 116. The flow rate of the gas is lower due to the resistance in the tubular path 116 than in the case the tubular path 116 is unpressed by the pushing member 140. The pressure in the balloon 74 therefore rises more slowly than in the case no resistance develops in the tubular path 116. Hence, the balloon 74 is slowly inflated, gradually expanding the intestine wall. When the pressure in the balloon 74 reaches an appropriate value, the doctor pushes the stop button 122, stopping the pump 114. Thus, the wall of the small intestine α is expanded slowly.

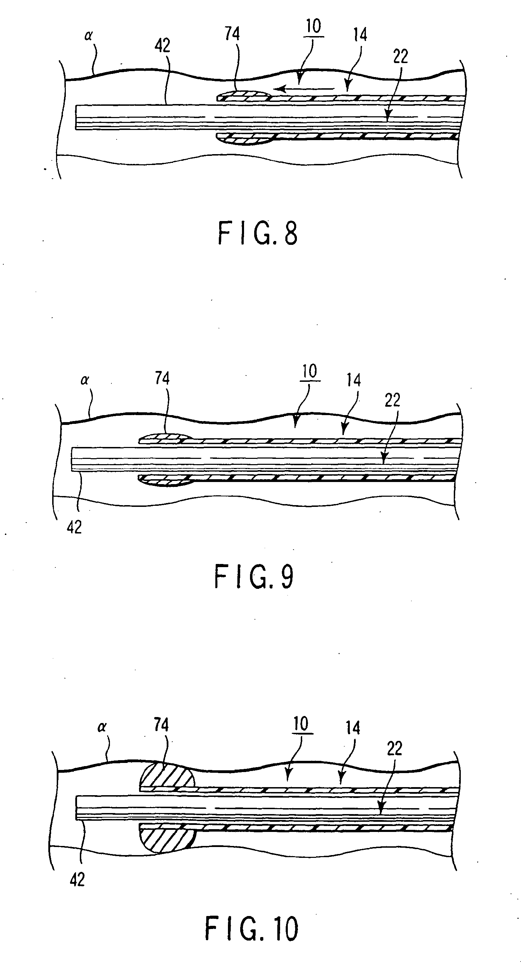

While the balloon 74 is retained on the inner surface of the small intestine α, the doctor pulls both the overtube 14 and the insertion section 22 toward the proximal end of the endoscope 12. The small intestine α is therefore and contracted. At this point, the bending part of the small intestine α is pulled as mentioned above and stretched. As a result, the insertion section 22 of the endoscope 12 can be easily inserted deeper into the small intestine α. The doctor then moves the insertion section 22 as deep as possible in the small intestine α, with respect to the overtube 14 (see FIG. 7). Then, the balloon 74 is deflated (see FIG. 8).

More precisely, the doctor pushes the depressurizing button 126 provided on the controller 120. When pushed, the depressurizing button 126 generates a signal. This signal is output from the remote controller 120 to the control circuit 118. Upon receiving the signal, the control circuit 118 drives the pump 114, which discharges gas. The control circuit 118 supplies the signal to the motor 138, and drives the motor 138. The driving shaft of the motor 138 rotates half around the axis of the driving shaft. The pushing member 140 is thereby rotated half around its axis and fixed in position. The space between the support member 142 and the pushing member 140 is thereby expanded. The tubular path 116 is no longer pressed. The tubular path 116 therefore expands by virtue of its elastic force. The gas can therefore be discharged by the pump 114 at a higher rate than in the case the tubular path 116 is pressed by the pushing member 140. Hence, the balloon 74 is deflated in a shorter time than it is inflated. Then, the doctor pushes the stop button 122, whereby the pump 114 stops operating. Since the balloon 74 is quickly deflated, the doctor can immediately start performing the next medical step.

Now that the balloon 74 has been deflated, the overtube 14 is moved toward the distal end of the insertion section 22, along the insertion section 22, as illustrated in FIG. 9. Thereafter, as shown in FIG. 10, the balloon 74 is inflated again until it contacts the intestine wall. The balloon 74 is therefore set in position in the small intestine α. Then, as described above, the overtube 14 guides the distal end of the insertion section 22 of the endoscope 12 deeper in the small intestine α.

As can be understood from the foregoing, this embodiment can achieve the following advantages.

To inflate the balloon 74 by supplying the gas into it from the pump 114 through the tubular path 116, the pushing member 140 can narrow the tubular path 116 to make it difficult for the gas to flow (can lower the flow rate of the gas). The balloon 74 can therefore be inflated slowly. Thus, a force can be exerted on the inner surface of the intestine, gradually expanding the intestine. The balloon 74 can be held in contact with the inner surface of the intestine, setting the overtube 14 at a specific position in the intestine. To deflate the balloon 74, the pushing member 140 is moved away from the tubular path 116, allowing the path 116 to have its sectional area increased to the initial value. The gas can then be discharged fast. This enables the doctor to start performing the next medical step at once.

The position of the pushing member 140 can be easily controlled merely by operating the remote controller 120. Hence, the power of the pump 114 can be minutely changed in accordance with the degree to which the balloon 74 has been inflated or deflated.

In the present embodiment, the pushing member 140 has a substantially elliptical cross section and has a rotation axis passing a point deviating from the center (set at, for example, the focus of the ellipsis). Instead, the rotation axis of the member 140 may pass the center. In this case, the pushing member 140 can press and release the tubular path 116 as it is rotated a quarter (1/4) around its axis.

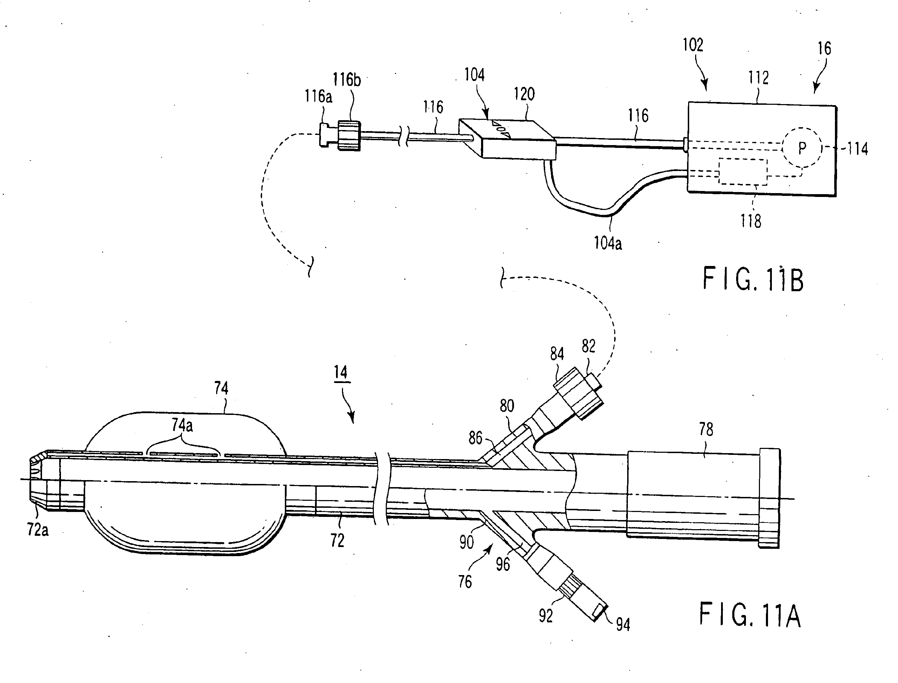

A second embodiment of this invention will be described, with reference to FIGS. 11A to 12B. This embodiment is a modification of the first embodiment. The components identical to those of the first embodiment are designated by the same reference numbers and will not be described in detail. The endoscope 12 is identical in configuration to the endoscope shown in FIG. 1, and the overtube 14 is similar in configuration to its counterpart of the first embodiment as can be seen from FIG. 11A.

As shown in FIG. 11B, the fluid-supplying/discharging system 16 according to the second embodiment includes a supplying/discharging device 102 and a tubular-path-resistance changing mechanism 104. A remote controller 120 for controlling the supplying/discharging device 102 is provided on the tubular-path-resistance changing mechanism 104.

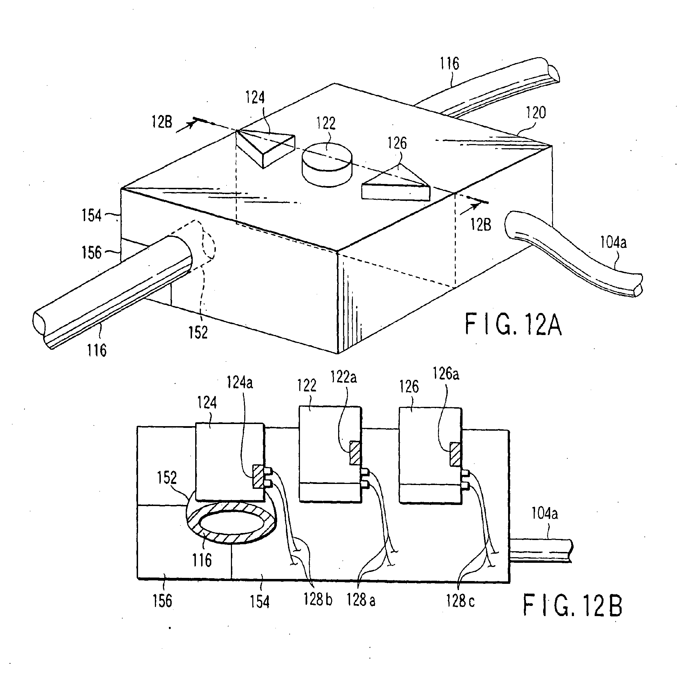

As shown in FIGS. 12A and 12B, the tubular-path-resistance changing mechanism 104 includes a tubular-path receptacle 152 for holding the tubular path 116, in addition to the remote controller 120. The tubular-path receptacle 152 is constituted by a mechanism body 154 and a cover member 156. The cover member 156 is secured to the mechanism body 154 by means of click coupling. The cover member 156 can therefore be detached from the mechanism body 154. Hence, the tubular path 116 can easily attached to, and detached from, the tubular-path-resistance changing mechanism 104. The cover member 156 may be, if desired, hinged to the tubular-path-resistance changing mechanism 104, not by click coupling.

The remote controller 120 includes a stop button 122, a pressurizing button 124, and a depressurizing button 126, all provided on the mechanism body 154. As shown in FIG. 12B, the tubular-path receptacle 152 is arranged below the pressurizing button 124. When the pressurizing button 124 is pushed, the tubular path 116 is deformed.

The stop button 122, pressurizing button 124 and depressurizing button 126 are electrically connected to the control circuit 118 by a cable 104a that has one end arranged in the mechanism body 154. When pushed, the stop button 122, pressurizing button 124 and depressurizing button 126 are electrically connected to conductors 128a, 128b and 128c, respectively, which extend through the cable 104a and which are electrically connected to the control circuit 118.

How the endoscope apparatus 10 according to the second embodiment operates will be explained.

When pushed, the pressurizing button 124 pushes the tubular path 116. At the same time, the electrical contact 124a of the pressurizing button 124 is electrically connected to the conductor 128b extending through the cable 104a. The control circuit 118 therefore drives the pump 114 as the pressurizing button 124 is pushed. Gas is thereby supplied through the tubular path 116 into the balloon 74, inflating the balloon 74. At this point, the pressurizing button 124 depresses the tubular path 116 in part, narrowing the tubular path 116. The flow of the gas is impaired at the depressed part of the tubular path 116. The gas therefore flows slowly, and the flow rate of the gas decreases. Hence, the balloon 74 is inflated more slowly than in the case where the pressurizing button 124 does not depress the tubular path 116 at all.

When the pressurizing button 124 is released, the tubular path 116 expands by virtue of its elastic force, back to its initial state. The cross-section area of the path 116 therefore increases. The electrical contact 124a of the pressurizing button 124 is electrically disconnected from the conductor 128b extending through the cable 104a. As a result, the pump 114 stops operating.

When the stop button 122 is pushed, the electrical contact 122a of the stop button 122 is electrically connected to the conductor 128a extending through the cable 104a. The control circuit 118 therefore stops driving the pump 114 as the stop button 122 is pushed. The control circuit 118 stops the pump 114 even if the pressurizing button 124 is pushed at the same time the stop button 122 is pushed.

When the depressurizing button 126 is pushed, the electrical contact 126a of the depressurizing button 126 is electrically connected to the conductor 128c extending through the cable 104a. The control circuit 118 therefore drives the pump 114 as the depressurizing button 126 is pushed. If the pressurizing button 124 is pushed at the same time the depressurizing button 126 is pushed, the control circuit 118 drives the pump 114 such that the pressure in the tubular path 116 is lowered.

As can be understood from the foregoing, the second embodiment can achieve the following advantages.

Depression of the pressurizing button 124 can not only change the cross-sectional area of the tubular path 116, but also control the operation of the pump 114. Since the cross-sectional area of the tubular path 116 can thus be controlled, the flow rate of the gas can be controlled. Hence, the balloon 74 can be slowly inflated. The speed with which the balloon 74 is inflated can be adjusted by depressing the tubular path 116 to a desired degree.

Further, if the stop button 122 or the depressurizing button 126 is pushed while the pressurizing button 124 remains pushed, the pump 114 will be stopped or will be driven to lower the pressure in the tubular path 116.

Since the tubular path 116 is arranged in the tubular-path receptacle 152, only the cover member 156 can be opened with respect to the mechanism body 154. This makes it easy to replace the tubular path 116 with a new one.

An O-ring, for example, may be provided at the end of the tubular-path receptacle 152 of the tubular-path-resistance changing mechanism 104. In this case, liquid or the like can be prevented from flowing into the tubular-path receptacle 152. The tubular-path-resistance changing mechanism 104 can therefore be used repeatedly.

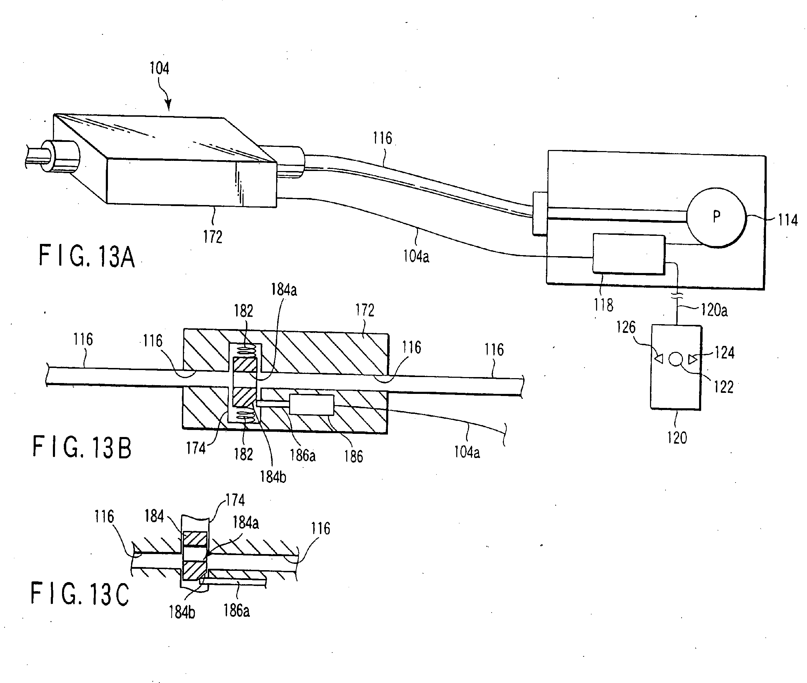

A third embodiment of the present invention will be described, with reference to FIGS. 13A to 13C. This embodiment is a modification of the first embodiment. The components identical to those of the first embodiment are designated by the same reference numbers and will not be described in detail.

As shown in FIGS. 13A and 13B, the tubular-path-resistance changing mechanism 104 includes a housing 172 having a tubular path 116. The housing 172 has a space 174 that extends, for example, vertically as shown in FIG. 13B. This tubular-path-resistance changing mechanism 104 has a pair of springs 182 and one movable body 184, which are provide in the space 174. The springs 182 are provided at the ends of the movable body 184, respectively. One spring 182 is interposed between the upper end of the space 174 and the upper end of the movable body 184. The other spring 182 is interposed between the lower end of the space 174 and the lower end of the moving body 184. Supported by the springs 182, the body 184 can move up and down in the space 174.

The movable body 184 has a communication path 184a shaped like, for example, a column. The movable body 184 is shaped like, for example, a rectangular parallelepiped. The movable body 184 has an inclined face 184b at a lower corner. The face 184b inclines at, for example, 45° to the axis of the tubular path 116. The angle at which the face 184b inclines may be changed, if necessary, in accordance with the distance the movable body 184 should be moved.

A linear motor 186 is provided in the housing 172 of the tubular-path-resistance changing mechanism 104. The drive shaft 186a of the linear motor 186 extends parallel to the axis of the tubular path 116. The drive shaft 186a can move to have its distal end move into and out of the space 174. The distal end of the drive shaft 186a abuts on the inclined face 184b of the movable body 184. Therefore, the movable body 184 moves upwards in the space 174 when the drive shaft 186a of the linear motor 186 pushes the inclined face 184b, and moves downwards by its own weight and by virtue of the action the upper spring 182 when the drive shaft 186a of the linear motor 186 stop pushing the inclined face 184b. As shown in FIG. 13C, in the normal state (that is, while the drive shaft 186a of the motor 186 is moved to its distal end into the space 174), the axis of the communication path 184a somewhat deviates from that of the tubular path 116. Hence, an abrupt inflation of the balloon 74 is prevented even if the pressurizing button 124 is pushed.

How the endoscope apparatus 10 according to the third embodiment operates will be explained.

When the stop button 122 on the remote controller 120 is pushed, the tubular path 116 and the communication path 184a of the movable body 184 axially deviate from each other (assuming the normal state) as shown in FIG. 13C. When the pressurizing button 124 is pushed, it generates a signal. The signal is input to the control circuit 118. Upon receiving the signal, the control circuit 118 supplies power via the cable 104a to the linear motor 186, driving the motor 186. The drive shaft 186a of the motor 186 moves, pushing the inclined face 184b of the movable body 184. The movable body 184 shown in FIG. 13C therefore moves up and its communication path 184a more deviates from that of the tubular path 116. Then, the flow rate of the gas decreases. Thereafter, the pump 114 is operated, supplying the gas into the balloon 74 of the overtube 14 through the tubular path 116 and communication path 184a. Since the flow rate of the gas is low, because of the axial deviation of the paths 116 and 184a, the gas is supplied at low speed, and the balloon 74 is inflated slowly.

When the stop button 122 is pushed, the control circuit 118 stops the pump 114. Further, the control circuit 118 drives the linear motor 186, pulling the drive shaft 186a from the inclined face 184b of the movable body 184. The movable body 184 shown in FIG. 13C therefore moves downwards, making the communication path 184a assume the normal positional relation with the tubular path 116.

When the depressurizing button 126 is pushed, the control circuit 118 drives both the pump 114 and the linear motor 186. Then, the drive shaft 186a of the motor 186 is pulled as shown in FIG. 13B, no long pushing the inclined face 184b of the movable body 184. In this case, the tubular path 116 and the communication path 184a almost come into axial alignment. As a result, the gas flows quickly from the balloon 74, whereby the balloon 74 is quickly deflated.

As can be understood from the foregoing, the third embodiment can achieve the following advantages.

As the movable body 184 is moved up or down in the space 174, the axial alignment between the tubular path 116 and the communication path 184a can be varied. To inflate the balloon 74, the axial alignment is decreased so that the flow rate of the gas being supplied into the balloon 74 is lowered. The balloon 74 can therefore be inflated slowly. To deflate the balloon 74, the axial alignment is increased so that the flow rate of the gas is raised. The balloon 74 can therefore be deflated quickly.

In the present embodiment, the movable body 184 takes one position (normal position) when the stop button 122 is pushed, and another position when the pressurizing button 124 is pushed. Instead, the normal position may be the position that the movable body 184 assumes when pressurizing the button 124.

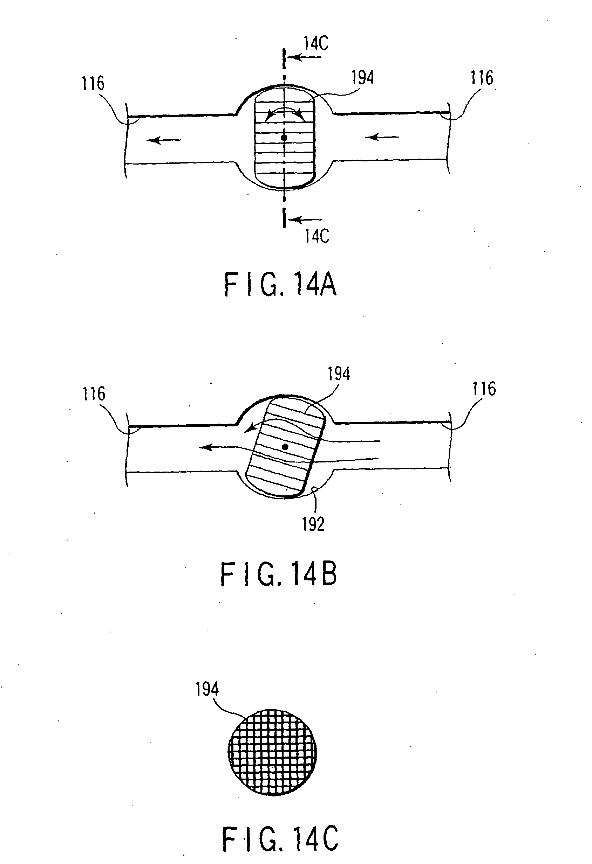

An example useful for understanding the present invention will be described with reference to FIGS. 14A to 14C. This example is a modification of the third embodiment.

As shown in FIG. 14A, the tubular path 116 has a space 192. A filter 194 (see FIG. 14C) is provided in the space 192 and can rotate. The filter 194 adjusts the flow rate of gas in accordance with the angle through which it has rotated. FIG. 14A shows the filter 194 positioned to allow the passage of the gas well. While the filter 194 stays in this position, the flow rate of the gas scarcely changes. FIG. 14B shows the filter 194 positioned to suppress the flow of the gas. While the filter 194 remains in this position, the flow rate and flow speed of gas greatly changes after the gas passes through the filter 194.

The filter 194 is mounted on the shaft (drive shaft) of a motor (not shown) and is connected to the control circuit 118. The angle by which the filter 194 is rotated can therefore be changed as needed.

When the stop button 122 or the pressurizing button 124 provided on the remote controller 120 is pushed, the filter 194 is rotated as shown in FIG. 14B. Therefore, when the pressurizing button 124 is pushed, the gas flows at low rate though the pump 114 is driven. As a result, the balloon 74 is inflated slowly.

When the depressurizing button 126 is pushed, the filter 194 is rotated, assuming the position shown in FIG. 14A. Therefore, the pump 114 discharges the gas from the balloon 74 at a high flow rate. As a result, the balloon 74 is deflated quickly.

In any embodiment and in the example described above, the endoscope apparatus 10 is used to examine the small intestine α. Nevertheless, the endoscope apparatus 10 can be inserted into the large intestine through the anus to examine the large intestine in the same way as to examine the small intestine. If the endoscope apparatus 10 is used to examine any other organ, the balloon may preferably be inflated faster than it is deflated.

In such a case, the pushing member 140 takes the position shown in FIG. 3A in, for example, the first embodiment, while the pump 114 remains not used. To inflate the balloon 74, the tubular path 116 is set to the state shown in FIG. 3A. The balloon 74 is therefore inflated quickly. To deflate the balloon 74, the tubular path 116 is set to the state shown in FIG. 3B. Thus, the balloon 74 is deflated slowly. Not only the first embodiment, but also the second and third embodiments and the example operate in this mode.

The fluid-supplying/discharging system 16 may be coupled not only to the overtube 14, but also to any other medical device that supplies and discharges fluid.

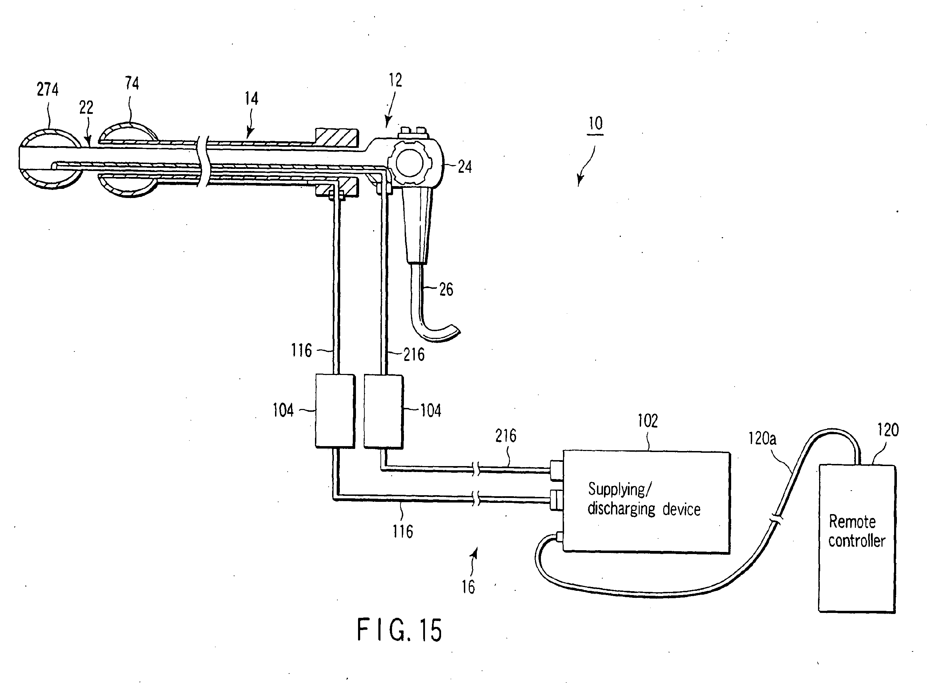

A fourth embodiment of the present invention will be described with reference to FIG. 15. This embodiment is a modification of any one of the first to third embodiments.

As shown in FIG. 15, this embodiment has a balloon 274, in addition to the balloon 74 mounted on the tubular body 72. The balloon 274 is mounted on the distal end of the insertion section 22 of the endoscope 12. A tubular path (communication path) 216 connects the balloon 274 to the fluid-supplying/discharging system 16. Like the balloon 74 used in the first to third embodiments, the balloon 274 is inflated slowly and deflated quickly.

Therefore, the insertion section 22 of the endoscope 12 can be inserted deeper into the intestine, with the overtube 14 set at a specific position with respect to the intestine wall. In addition, the distal end of the overtube 14 can be guided deeper into a body cavity, with the insertion section 22 set in a specific position with respect to the intestine wall.

## Claims

1. A fluid-supplying/discharging system (16) for use in medical devices, comprising:
a supplying/discharging device (102) including:
a pump (114) capable of supplying and discharging fluid;
a tubular path (116) having two ends, connected at one end to the pump and at the other end to the medical device (14); and
a control device (118) connected to the pump and configured to control the supplying and discharging of the fluid to and from the pump; and
a tubular-path-resistance changing mechanism (104) configured to change resistance to the fluid flowing through the tubular path;
wherein the pump (114) is configured to supply the fluid toward the tubular path (116) at a substantially constant speed and to discharge the fluid through the tubular path at a substantially constant speed; and
the tubular-path-resistance changing mechanism (104) includes a cross-sectional-area changing section (140, 142; 124, 152; 182, 184, 186), the cross-sectional-area changing section (140, 142; 124, 152; 182, 184, 186) in use decreasing a cross-sectional area of at least one part of the tubular path during fluid supply, from a maximum value, thereby exerting resistance to the fluid flowing through the tubular path and lowering a flow rate of the fluid at the other end of the tubular path more than at the one end thereof, and in use increasing the cross-sectional area of the at least one part of the tubular path during fluid discharge, to the maximum value or a value close thereto, thereby enabling a faster discharge of the fluid than supply of the fluid, or the cross-sectional-area changing section (140, 142; 124, 152; 182, 184, 186) in use increasing a cross-sectional area of at least one part of the tubular path during fluid supply, to a maximum value or a value close thereto, thereby enabling a faster supply of the fluid than discharge of the fluid, and exerting resistance to the fluid flowing through the tubular path and lowering a flow rate of the fluid at the other end of the tubular path more than at the one end thereof, and in use decreasing the cross-sectional area of the at least one part of the tubular path during fluid discharge, from the maximum value, thereby lowering a speed at which the fluid is discharged from the tubular path;
**characterized in that**
the tubular path (116) has such elasticity to have the cross-sectional area decreased from the maximum value when pressed from outside and increased back to the maximum value or a value close thereto when released from a pressure applied externally; and
the cross-sectional-area changing section (140, 142; 124, 152) has a pushing unit (140; 124) which in use pushes the tubular path to decrease the cross-sectional area of the tubular path from the maximum value and which in use moves away from the tubular path to increase the cross-sectional area of the tubular path back to the maximum value or a value close thereto.

2. The fluid-supplying/discharging system (16) according to claim 1, **characterized in that**
the pushing unit (140) includes a motor (138) which is configured to control an angle of rotation and a cam (140) which rotates as a drive shaft of the motor is driven, and
the motor is electrically connected to the control device (118) which is configured to control the pump (114), too, so that the drive shaft of the motor is driven as the pump is driven.

3. A fluid-supplying/discharging system (16) for use in medical devices, comprising:
a supplying/discharging device (102) including:
a pump (114) capable of supplying and discharging fluid;
a tubular path (116) having two ends, connected at one end to the pump and at the other end to the medical device (14); and
a control device (118) connected to the pump and configured to control the supplying and discharging of the fluid to and from the pump; and
a tubular-path-resistance changing mechanism (104) configured to change resistance to the fluid flowing through the tubular path;
wherein the pump (114) is configured to supply the fluid toward the tubular path (116) at a substantially constant speed and to discharge the fluid through the tubular path at a substantially constant speed; and
the tubular-path-resistance changing mechanism (104) includes a cross-sectional-area changing section (140, 142; 124, 152; 182, 184, 186), the cross-sectional-area changing section (140, 142; 124, 152; 182, 184, 186) in use decreasing a cross-sectional area of at least one part of the tubular path during fluid supply, from a maximum value, thereby exerting resistance to the fluid flowing through the tubular path and lowering a flow rate of the fluid at the other end of the tubular path more than at the one end thereof, and in use increasing the cross-sectional area of the at least one part of the tubular path during fluid discharge, to the maximum value or a value close thereto, thereby enabling a faster discharge the fluid than supply of the fluid, or the cross-sectional-area changing section (140, 142; 124, 152; 182, 184, 186) in use increasing a cross-sectional area of at least one part of the tubular path during fluid supply, to a maximum value or a value close thereto, thereby enabling a faster supply of the fluid than discharge of the fluid, and exerting resistance to the fluid flowing through the tubular path and lowering a flow rate of the fluid at the other end of the tubular path more than at the one end thereof, and in use decreasing the cross-sectional area of the at least one part of the tubular path during fluid discharge, from the maximum value, thereby lowering a speed at which the fluid is discharged from the tubular path;
**characterized in that**
the tubular-path-resistance changing mechanism (182, 184, 186) is mounted on the tubular path (116) and has a deviation unit having a fluid communication path (184a), wherein the deviation unit in use deviates an axis of the fluid communication path (184a) from an axis of the tubular path, thereby changing the resistance to the fluid flowing through the tubular path.

4. An endoscope apparatus (10) comprising:
a fluid-supplying/discharging system (16) according to any of claims 1 to 3;
a tubular body (72) having a part for guiding an insertion section of an endoscope and a communication path (74) connected to the other end of the tubular path and communicating with the tubular path; and
a balloon (74) mounted on the tubular body, communicating with the communication path and configured to be inflated and deflated.

5. An endoscope apparatus (10) comprising:
an endoscope (12) including:
an insertion section (22), and
an operation section (24) to be inserted into a body cavity;
a fluid-supplying/discharging system (16) according to any of claims 1 to 3;
a tubular body (72) having a part for guiding an insertion section of an endoscope and a communication path (74) connected to the other end of the tubular path and communicating with the tubular path; and
a balloon (74) mounted on the tubular body, communicating with the communication path and configured to be inflated and deflated.

6. The endoscope apparatus (10) according to claim 5, **characterized in that** the insertion section (22) of the endoscope (12) has another balloon (274) capable of being inflated and deflated.

## Patentansprüche

1. Fluidzuführ-/-abgabesystem (16) zur Verwendung in medizinischen Geräten, umfassend:
ein Zuführ-/Abgabegerät (102) enthaltend:
eine Pumpe (114), die dazu geeignet ist, Fluid zuzuführen und abzugeben;
einen schlauchförmigen Pfad (116) mit zwei Enden, der an einem Ende mit der Pumpe und an dem anderen Ende mit dem medizinischen Gerät (14) verbunden ist; und
ein Steuergerät (118), das mit der Pumpe verbunden ist und dazu eingerichtet ist, das Zuführen und Abgeben des Fluids zu und von der Pumpe zu steuern; und
einen Mechanismus (104) zum Ändern des Widerstands des schlauchförmigen Pfads (104), der dazu eingerichtet ist, den Widerstand auf das durch den schlauchförmigen Pfad fließende Fluid zu verändern;
wobei die Pumpe (114) dazu eingerichtet ist, das Fluid dem schlauchförmigen Pfad (116) bei einer im Wesentlichen konstanten Geschwindigkeit zuzuführen und das Fluid durch den schlauchförmigen Pfad bei einer im Wesentlichen konstanten Geschwindigkeit abzugeben; und
der Mechanismus (104) zum Ändern des Widerstands des schlauchförmigen Pfads einen Abschnitt (140, 142; 124, 152; 182, 184, 186) zum Ändern einer Querschnittsfläche aufweist, wobei der Abschnitt (140, 142; 124, 152; 182, 184, 186) zum Ändern einer Querschnittsfläche bei Benutzung eine Querschnittsfläche von zumindest einem Teil des schlauchförmigen Pfads während Fluidzufuhr von einem maximalen Wert verringert, um dadurch einen Widerstand auf das durch den schlauchförmigen Pfad fließende Fluid auszuüben und eine Durchflussgeschwindigkeit des Fluids an dem anderen Ende des schlauchförmigen Pfads mehr als an dem einen Ende davon herabzusetzen, und bei Benutzung die Querschnittsfläche von dem zumindest einen Teil des schlauchförmigen Pfads während Fluidsabgabe auf den maximalen Wert oder einen dazu nahen Wert vergrößert, um dadurch eine schnellere Abgabe des Fluids als Zufuhr des Fluids zu ermöglichen, oder wobei der Abschnitt (140, 142; 124, 152; 182, 184, 186) zum Ändern einer Querschnittsfläche bei Benutzung eine Querschnittsfläche von zumindest einem Teil des schlauchförmigen Pfads während Fluidzufuhr auf einen maximalen Wert oder einen dazu nahen Wert vergrößert, um dadurch eine schnellere Zufuhr des Fluids als Abgabe des Fluids zu ermöglichen, und einen Widerstand auf das durch den schlauchförmigen Pfad fließende Fluid auszuüben und eine Durchflussgeschwindigkeit des Fluids an dem anderen Ende des schlauchförmigen Pfads mehr als an dem einen Ende davon herabzusetzen, und bei Benutzung die Querschnittsfläche von dem zumindest einen Teil des schlauchförmigen Pfads während Fluidabgabe von dem maximalen Wert verringert, um dadurch eine Geschwindigkeit, bei welcher das Fluid von dem schlauchförmigen Pfad abgegeben wird, herabzusetzen;
**dadurch gekennzeichnet, dass**
der schlauchförmige Pfad (116) eine derartige Elastizität aufweist, dass die Querschnittsfläche von dem Maximalwert verringert wird, wenn von außen gedrückt wird, und zurück auf den Maximalwert oder auf einen dazu nahen Wert vergrößert wird, wenn er von einem extern angelegten Druck befreit wird; und
der Abschnitt (140, 142; 124, 152) zum Ändern der Querschnittsfläche eine Druckeinheit (140; 124) aufweist, welche bei Benutzung den schlauchförmigen Pfad drückt, um die Querschnittsfläche des schlauchförmigen Pfads von dem maximalen Wert zu verringern und welche bei Benutzung sich weg von dem schlauchförmigen Pfad bewegt, um die Querschnittsfläche des schlauchförmigen Abschnitts zurück auf den Maximalwert oder einen dazu nahen Wert zu vergrößern.

2. Fluidzuführ-/-abgabesystem (16) nach Anspruch 1, **dadurch gekennzeichnet, dass**
die Druckeinheit (140) einen Motor (138), welcher dazu eingerichtet ist, einen Drehwinkel zu steuern, und einen Nocken (140) aufweist, welcher sich dreht, wenn eine Antriebsachse des Motors bewegt wird, und
der Motor elektrisch mit dem Steuergerät (118) verbunden ist, welches dazu eingerichtet ist, die Pumpe (114) auch derart zu steuern, dass die Antriebsachse des Motors angetrieben wird, wenn die Pumpe angetrieben wird.

3. Fluidzuführ-/-abgabesystem (16) zur Verwendung in medizinischen Geräten, umfassend:
ein Zuführ-/Abgabegerät (102) enthaltend:
eine Pumpe (114), die dazu geeignet ist, Fluid zuzuführen und abzugeben;
einen schlauchförmigen Pfad (116) mit zwei Enden, der an einem Ende mit der Pumpe und an dem anderen Ende mit dem medizinischen Gerät (14) verbunden ist; und
ein Steuergerät (118), das mit der Pumpe verbunden ist und dazu eingerichtet ist, das Zuführen und Abgeben des Fluids zu und von der Pumpe zu steuern; und
einen Mechanismus (104) zum Ändern des Widerstands des schlauchförmigen Pfads, der dazu eingerichtet ist, den Widerstand auf das durch den schlauchförmigen Pfad fließende Fluid zu verändern;
wobei die Pumpe (114) dazu eingerichtet ist, das Fluid dem schlauchförmigen Pfad (116) bei einer im Wesentlichen konstanten Geschwindigkeit zuzuführen und das Fluid durch den schlauchförmigen Pfad bei einer im Wesentlichen konstanten Geschwindigkeit abzugeben; und
der Mechanismus (104) zum Ändern des Widerstands des schlauchförmigen Pfads einen Abschnitt (140, 142; 124, 152; 182, 184, 186) zum Ändern einer Querschnittsfläche aufweist, wobei der Abschnitt (140, 142; 124, 152; 182, 184, 186) zum Ändern einer Querschnittsfläche bei Benutzung eine Querschnittsfläche von zumindest einem Teil des schlauchförmigen Pfads während Fluidzufuhr von einem maximalen Wert verringert, um dadurch einen Widerstand auf das durch den schlauchförmigen Pfad fließende Fluid auszuüben und eine Durchflussgeschwindigkeit des Fluids an dem anderen Ende des schlauchförmigen Pfads mehr als an dem einen Ende davon herabzusetzen, und bei Benutzung die Querschnittsfläche von dem zumindest einen Teil des schlauchförmigen Pfads während Fluidabgabe auf den maximalen Wert oder einen dazu nahen Wert vergrößert, um dadurch eine schnellere Abgabe des Fluids als Zufuhr des Fluids zu ermöglichen, oder wobei der Abschnitt (140, 142; 124, 152; 182, 184, 186) zum Ändern einer Querschnittsfläche bei Benutzung eine Querschnittsfläche von zumindest einem Teil des schlauchförmigen Pfads während Flüssigkeitszufuhr auf einen maximalen Wert oder einen dazu nahen Wert vergrößert, um dadurch eine schnellere Zufuhr des Fluids als Abgabe des Fluids zu ermöglichen, und einen Widerstand auf das durch den schlauchförmigen Pfad fließende Fluid auszuüben und eine Durchflussgeschwindigkeit des Fluids an dem anderen Ende des schlauchförmigen Pfads mehr als an dem einen Ende davon herabzusetzen, und bei Benutzung die Querschnittsfläche des zumindest einen Teils des schlauchförmigen Pfads während Fluidabgabe von dem maximalen Wert verringert, um dadurch eine Geschwindigkeit, bei welcher das Fluid von dem schlauchförmigen Pfad abgegeben wird, herabzusetzen;
**dadurch gekennzeichnet, dass**
der Mechanismus (182, 184, 186) zum Ändern des Widerstands des schlauchförmigen Pfads an dem schlauchförmigen Pfad (116) angebracht ist und eine Abweichungseinheit mit einem Fluidkommunikationspfad (184a) aufweist, wobei die Abweichungseinheit bei Benutzung eine Achse des Fluidkommunikationspfads (184a) von einer Achse des schlauchförmigen Pfads ablenkt, um dadurch den Widerstand auf das durch den schlauchförmigen Pfad fließende Fluid zu verändern.

4. Endoskopvorrichtung (10) umfassend:
ein Fluidzuführ-/-abgabesystem (16) nach einem der Ansprüche 1 bis 3;
einen schlauchförmigen Körper (72) mit einem Teil zum Führen eines Einführabschnitts eines Endoskops und einem Kommunikationspfad (74), der mit dem anderen Ende des schlauchförmigen Pfads verbunden ist und mit dem schlauchförmigen Pfad kommuniziert;
einen an dem schlauchförmigen Körper angebrachten Ballon (74), der mit dem Kommunikationspfad kommuniziert und dazu eingerichtet ist, aufgeblasen und entleert zu werden.

5. Endoskopvorrichtung (10) umfassend:
ein Endoskop (12) enthaltend:
einen Einführabschnitt (22), und
einen Betätigungsabschnitt (24), der in einen Körperhohlraum einzuführen ist;
ein Fluidzuführ-/-abgabesystem (16) nach einem der Ansprüche 1 bis 3;
einen schlauchförmigen Körper (72) mit einem Teil zum Führen eines Einführabschnitts eines Endoskops und einem Kommunikationspfad (74), der mit dem anderen Ende des schlauchförmigen Pfads verbunden ist und mit dem schlauchförmigen Pfad kommuniziert; und
einen an dem schlauchförmigen Körper angebrachten Ballon (74), der mit dem Kommunikationspfad kommuniziert und dazu eingerichtet ist, aufgeblasen und entleert zu werden.

6. Endoskopvorrichtung (10) nach Anspruch 5, **dadurch gekennzeichnet, dass** der Einführabschnitt (22) des Endoskops (12) einen weiteren Ballon (274) aufweist, der dazu geeignet ist, aufgeblasen und entleert zu werden.

## Revendications

1. Système d'alimentation et d'évacuation en fluide (16) pour être utilisé dans des dispositifs médicaux, comprenant :
- un dispositif d'alimentation et d'évacuation (102) incluant :
- une pompe (114) capable d'alimenter et d'évacuer le fluide ;
- un passage tubulaire (116) ayant deux extrémités, reliées à une extrémité de la pompe et à l'autre extrémité à un dispositif médical (14) ; et
- un dispositif de commande (118) relié à la pompe et configuré pour commander l'alimentation et l'évacuation du fluide vers et en provenance de la pompe ; et
- un mécanisme de changement de la résistance du passage tubulaire (104) configuré pour changer la résistance au fluide passant à travers le passage tubulaire ;
- dans lequel la pompe (114) est configurée pour fournir le fluide vers le passage tubulaire (116) à une vitesse sensiblement constante et pour évacuer le fluide à travers le passage tubulaire à une vitesse sensiblement constante ; et
- le mécanisme de changement de la résistance du passage tubulaire (104) inclut une section de changement de l'aire de la section transversale (140, 142, 124, 152, 182, 184, 186), la section de changement de l'aire de la section transversale (140, 142, 124, 152, 182, 184, 186) en utilisation diminuant une aire de la section transversale d'au moins une partie du passage tubulaire durant l'alimentation en fluide, à partir d'une valeur maximale, exerçant ainsi une résistance au fluide passant à travers le passage tubulaire et diminuant le débit de fluide à l'autre extrémité du passage tubulaire plus qu'à l'une extrémité de celui-ci, et au cours de l'utilisation augmentant l'air de la section transversale de l'au moins une partie du passage tubulaire durant l'évacuation du fluide, jusqu'à une valeur maximale ou une valeur proche du maximum, permettant ainsi une évacuation plus rapide du fluide que l'alimentation en fluide, ou la section de changement de l'aire de la section transversale 140, 142, 124, 152, 182, 184, 186) au cours de l'utilisation augmentant une aire de la section transversale d'au moins une partie du passage tubulaire durant l'alimentation en fluide, à une valeur maximale ou une valeur proche du maximum, permettant ainsi une alimentation plus rapide en fluide que l'évacuation en fluide, et exerçant une résistance au fluide passant à travers le passage tubulaire et diminuant un débit du fluide à l'autre extrémité du passage tubulaire plus qu'à l'une extrémité de celui-ci, et en utilisation diminuant l'aire de la section transversale d'au moins une partie du passage tubulaire durant l'évacuation du fluide, à partir d'une valeur maximale, diminuant ainsi une vitesse à laquelle le fluide est évacué du passage tubulaire ;
**caractérisé en ce que**
- le passage tubulaire (116) a une élasticité permettant que l'aire de la section transversale diminue depuis une valeur maximale lorsqu'il est pressé depuis l'extérieur et réaugmente jusqu'à la valeur maximale ou à une valeur proche de celle-ci lorsque la pression appliquée extérieurement est relâchée.

2. Système d'alimentation et d'évacuation en fluide (16) selon la revendication 1, **caractérisé en ce que** l'unité de poussée (140) comprend un moteur (138) qui est configuré pour commander un angle de rotation et une came (140) qui tourne au fur et à mesure qu'un arbre d'entraînement du moteur est entraîné, et **en ce que** le moteur est relié électriquement au dispositif de commande (118) qui est configuré pour commander la pompe (114), également, de manière que l'arbre d'entraînement du moteur est entraîné au fur et à mesure que la pompe est entraînée.

3. Système d'alimentation et d'évacuation en fluide (16) pour être utilisé dans des dispositifs médicaux, comprenant :
- un dispositif d'alimentation et d'évacuation (102) incluant :
- une pompe (114) capable d'alimenter et d'évacuer le fluide ;
- un passage tubulaire (116) ayant deux extrémités, reliées à une extrémité de la pompe et à l'autre extrémité à un dispositif médical (14) ; et
- un dispositif de commande (118) relié à la pompe et configuré pour commander l'alimentation et l'évacuation du fluide vers et en provenance de la pompe ; et
- un mécanisme de changement de la résistance du passage tubulaire (104) configuré pour changer la résistance au fluide passant à travers le passage tubulaire ;
- dans lequel la pompe (114) est configurée pour fournir le fluide vers le passage tubulaire (116) à une vitesse sensiblement constante et pour évacuer le fluide à travers le passage tubulaire à une vitesse sensiblement constante ; et
- le mécanisme de changement de la résistance du passage tubulaire (104) inclut une section de changement de l'aire de la section transversale (140, 142, 124, 152, 182, 184, 186), la section de changement de l'aire de la section transversale (140, 142, 124, 152, 182, 184, 186) en utilisation diminuant une aire de la section transversale d'au moins une partie du passage tubulaire durant l'alimentation en fluide, à partir d'une valeur maximale, exerçant ainsi une résistance au fluide passant à travers le passage tubulaire et diminuant le débit de fluide à l'autre extrémité du passage tubulaire plus qu'à l'une extrémité de celui-ci, et au cours de l'utilisation augmentant l'air de la section transversale de l'au moins une partie du passage tubulaire durant l'évacuation du fluide, jusqu'à une valeur maximale ou une valeur proche du maximum, permettant ainsi une évacuation plus rapide du fluide que l'alimentation en fluide, ou la section de changement de l'aire de la section transversale 140, 142, 124, 152, 182, 184, 186) au cours de l'utilisation augmentant une aire de la section transversale d'au moins une partie du passage tubulaire durant l'alimentation en fluide, à une valeur maximale ou une valeur proche du maximum, permettant ainsi une alimentation plus rapide en fluide que l'évacuation en fluide, et exerçant une résistance au fluide passant à travers le passage tubulaire et diminuant un débit du fluide à l'autre extrémité du passage tubulaire plus qu'à l'une extrémité de celui-ci, et en utilisation diminuant l'aire de la section transversale d'au moins une partie du passage tubulaire durant l'évacuation du fluide, à partir d'une valeur maximale, diminuant ainsi une vitesse à laquelle le fluide est évacué du passage tubulaire ;
**caractérisé en ce que**
- le mécanisme de changement de la résistance du passage tubulaire (182, 184, 186) est monté sur le passage tubulaire (116) et a une unité de déviation ayant un passage de communication fluidique (184a), dans lequel l'unité de déviation en utilisation dévie un axe du passage de communication fluidique (184a) par rapport à un axe du passage tubulaire, permettant un changement de la résistance du fluide passant à travers le passage tubulaire.

4. Appareil d'endoscope (10) comprenant :
- un système d'alimentation/d'évacuation en fluide (16) selon l'une quelconque des revendications 1 à 3 ;
- un corps tubulaire (72) ayant une partie pour guider une section d'insertion d'un endoscope et un passage à communication (74) relié à l'autre extrémité du passage tubulaire et communiquant avec le passage tubulaire ; et
- un ballon (74) monté sur le corps tubulaire, communiquant avec le passage tubulaire et configuré pour être gonflé et dégonflé.

5. Appareil d'endoscope (10) comprenant :
- un endoscope (12) incluant :
- une section d'insertion (22), et
- une section d'opération (24) destinée à être insérée à l'intérieur d'une cavité du corps ;
- un système d'alimentation/d'évacuation en fluide (16) selon l'une quelconque des revendications 1 à 3 ;
- un corps tubulaire (72) ayant une partie pour guider une section d'insertion d'un endoscope et un passage à communication (74) relié à l'autre extrémité du passage tubulaire et communiquant avec le passage tubulaire ; et
- un ballon (74) monté sur le corps tubulaire, communiquant avec le passage tubulaire et configuré pour être gonflé et dégonflé.

6. Appareil d'endoscope (10) selon la revendication 5, **caractérisé en ce que** la section d'insertion (22) de l'endoscope (12) a un autre ballon (274) capable d'être gonflé et dégonflé.
